# EUROPEAN PATENT APPLICATION

(11) **EP 1 374 884 A1**
(43) Date of publication of application: **02.01.2004**
(21) Application number: 02707206.5
(22) Date of filing: 28.03.2002
(51) Int. Cl.: A61K 35/78, A61K 31/7048, A61K 31/353, A61K 31/35, A61P 39/02

(54) **PROTEOTOXIN NEUTRALIZER**

(30) Priority: 28.03.2001 JP 2001092303; 31.10.2001 JP 2001334722
(71) Applicant: ASAHI BREWERIES, LTD., Tokyo 104-0031 (JP)
(72) Inventor: TAGASHIRA, Motoyuki, c/o Asahi Breweries, Ltd., Moriya, Ibaraki 302-0106 (JP); IWAMARU, Yoshifumi, Chiba, Chiba 260-0808 (JP); NODA, Masatoshi, Yotsukaido, Chiba 284-0045 (JP); MIYAKE, Masami, Hanamigawa-ku, Chiba, Chiba-262-0026 (JP)
(74) Representative: Walter, Wolf-Jürgen
(86) International application number: PCT/JP2002/003046
(87) International publication number: WO 2002/078726

(57) **Abstract**

Proteotoxin neutralizers containing as the active ingredient proanthocyanidins obtained from hop.

## Description

### Industrial Field

The present invention relates to a proteotoxin neutralizer which contains proanthocyanidins obtained from hop as effective components.

### Background of the Art

Hop *(Humulus lupuls)* is a perennial plant of a Cannabaceae family, and the hop cone (a ripe unfertilized female flower) is generally called hop. The hop comprises, in addition to the cones, leaves, bines, roots and the like. A lupulin part (a yellow granule formed in the root of internal bracts of the hop cone) existing in the hop cone is a source of bitterness and perfume, and it is an important beer material along with yeast and malt in beer brewing. The hop can be used as a sedative drug and an anti-aphrodisiac in folk remedies. The hop bracts are formed by removing the lupulin part from the hop cone, and these bracts are useless. If circumstances require, these bracts are removed in beer brewing to be byproducts. In this case, the hop bracts are used as fertilizer. However, since special effective use is not found, it is hoped to develop a method having a high additional value for using the bracts.

In Japanese Patent Laid-open publication No. 09-2971, Japanese Patent Laid-open publication No. 09-163969, Japanese Patent Laid-open publication Nos. 09-295944, 10-25232, 2000-327582 and 2001-39886 (the applicant's application), it is recognized that hop, particularly the polyphenols derived from hop bracts have antioxidant action, sparkle-stabilizing action of sparkling malt drinks, anticaries action, deodorant action, antimetastatic action of cancer cells, and topoisomeraze-inhibiting action.

However, as to proanthocyanidins obtained from hop, no cases proving neutralization effect of proteotoxin have been found hitherto. At this point, bacterial toxin produced with virulent fungi such as cholera toxin produced with a cholera germ and vero toxin produced with enterohemorrhagic colibacillus such as 0-157 corresponds to the proteotoxin. Venom of snakes such as habus and vipers, plant toxin such as lysine corresponds to the proteotoxin. When the toxin itself is proteotoxin, the toxin is not limited by the above toxin. Problems to be settled by the invention

According to a report of WHO, infection diseases cause the death of 20 million all over the world. In spite of great development of medical treatment techniques, infection diseases are one of intimidation to humankind.

Among infection diseases such as cholera, pertussis, diphtheria, diarrhea caused by toxigenic Echerichia coli and opportunistic infection caused by Pseudomonas aeruginosa, it is known that each virus produces exotoxin having ADP-ribosyltransferase action, the exotoxin modifies a functional protein in vivo to lose the original function, and diseases are expressed. Namely, cholera toxin produced from cholera bacillus, pertussis toxin produced from pertussis bacillus, thermolabile enteric toxin (LT) produced from toxigenic E. coli, exotoxin produced from Psudomonas aeruginosa, botulinum C2 toxin produced from *Clostridium botulinum,* iota toxin produced from *Clostridium perfringenus* and the like are proteotoxin (ADP-ribosylation toxin) having ADP-ribosyltransferase action.

ADP-ribosyltransferase action means that nicotinamid is cut from NAD of intravital coenzyme and the remaining ADP-ribose part is irreversibly transferred to target protein. The target protein then irreversibly loses the intravital function. As an example, it is known that diphtheria toxin and exotoxin A from Pseudomonas aeruginosa ADP-ribosylate a peptide elongation factor EF-2, and cholera toxin and LT of toxigenic E. coli ADP-ribosylate Gsα.

Many peoples all over the world suffer from infectious diseases caused by pathogen factor of ADP-ribosylation toxin. According to a WHO report, the number of peoples suffered from cholera amounts to hundred thousands per year. Namely, peoples are exposed to the menace of the infectious diseases caused by pathogen factor of ADP-ribosylation toxin.

On the other hand, in dysentery, haemorrhagic colitis caused by vero toxin production E. coli (VTEC) and the like, each etiologic bacillus produces exotoxin having RNA N-glycosidase action, the exotoxin modifies a functional protein in vivo to lose the original function, and diseases are expressed. Namely, Shiga toxin produced from shigella dysenteriae, vero toxin (or it is called Shiga-like toxin) produced from VETC and the like are proteotoxin having RNA N-glycosidase action (RNA N-glycosylation toxin).

The RNA N-glycosidase action means that an N-glycoside bond of RNA is enzymically hydrolyzed. The proteotoxin having such action modifies ribosomal RNA in a ribosome that is a proteosynthesis device of cells to be irreversibly inactivated. Thereby, it is known that the proteiosynthesis of cells is stopped to develop the toxicity. As an example, Shiga toxin, vero toxin and the like specifically hydrolyze an N-glycoside bond of adenosine that is 4324^{th} from 5' end of 28S ribosomal.

Among infection diseases caused by RNA N-glycosilated toxin, infection diseases caused by VTEC occur frequently in the world regardless of developed countries and developing countries. Particularly, when the vero toxin transits into blood, since hemolytic uremic syndrome (HUS) or encephalopathy is affected to die or have sequellae, the infection diseases are serious. In Japan, it remains in people's memory that unprecedented food poisoning occurred in Osaka and over 6000 peoples were infected in 1996.

At the present time, antibiotics having antibiotic action to pathogenic germs are generally used for treating the infection diseases caused by ADP-ribosylation toxin or RNA N-glycosylation toxin. However, since stronger pathogenic germs tolerant to the antibiotics appear by administration of the antibiotics, it causes trouble at practical medical fields. Accordingly, it needs to develop a new method for treating without the antibiotics. Particularly, in case of the infection disease of VTEC, it is said that massive vero toxin is produced by VTEC due to the administration of antibiotics, thereby the symptom becomes serious *(Matsushiro et al., J. Bacteriol.,* 181, 2257-2260 (1999)). Accordingly, there are many opinions to deny the administration of antibiotics. Namely, in fact, it is asked for establishing urgently a new medical method in which materials such as antibiotics killing germs are not used.

Considering such conditions, the inventors of the present invention have tried to find a factor for neutralizing and detoxifying the proteotoxin produced from the germs causing the infection diseases, and thereby to resolve the problems. When effective factor for neutralizing the proteotoxin, particularly, effective factor for neutralizing the proteotoxin having ADP-ribosyltransferase action and RNA N-glycosidase action is found, there are meaningful in medical and industrial fields.

### Disclosure of Invention

The inventors of the present invention earnestly have studied and found that a kind of polyphenols in a hop detoxifies effectively ADP-ribosyltransferase action and RNA N-glycosidase action, and completed the present invention. The polyphenol is contained in hop stalk and bract parts, especially contained massively in the bract parts. The polyphenol is adsorbed to a resin showing affinity with polyphenol such as styrene-divinylbenzene resin. When the polyphenol is treated with a membrane having fraction molecules of 1000 or more, it shows a property that it does not penetrate the membrane. The polyphenol is a proanthocyanidin producing cyanidin when the polyphenol is heated and hydrolysed in an alcohol solution containing about 5% hydrochloric acid.

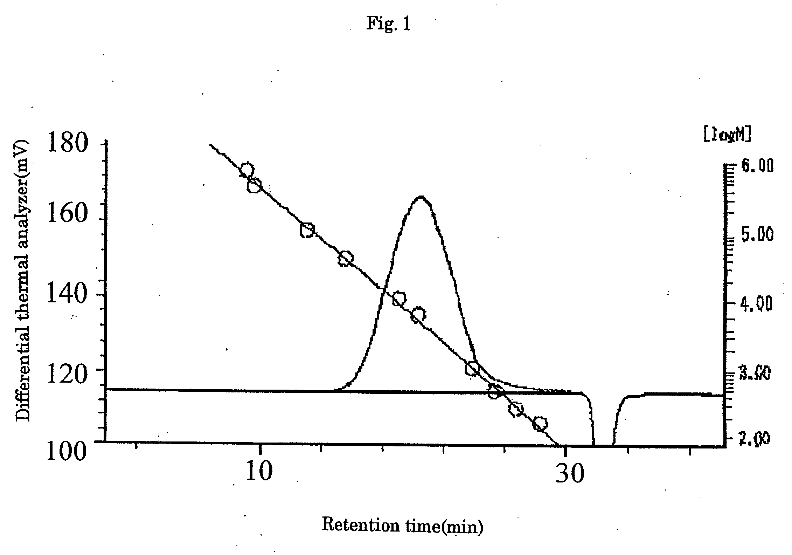

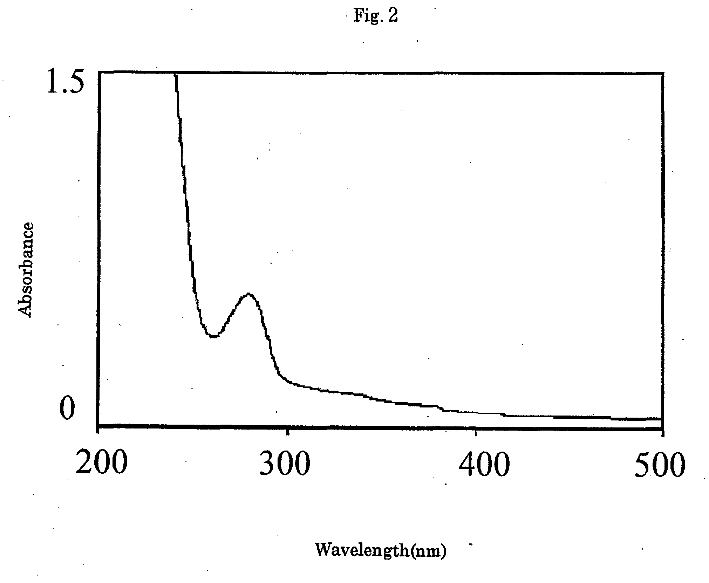

The proanthocyanidin gives a chromatogram of Fig. 1 in GPC (a gel penetration chromatogram) analysis and absorbance distribution of Fig. 2 in absorbance analysis.
The present invention relates to a proteotoxin neutralizer that contains as an effective component the proanthocyanidins contained in hop, preferably in a hop bract part.

### Brief Description of Drawings

Fig. 1 shows GPC (gel penetration chromatography) analysis result of proanthocyanidins derived from hop.

Fig. 2 shows absorbance distribution of proanthocyanidins derived from hop.

Fig. 3 shows HPLC analysis result of proanthocyanidins derived from hop.

Fig. 4 shows result of Example 12. The longitudinal axis shows radiation activity. The control shows experiment result that cholera toxin is not added. The height of a column is a mean value of experiment values (n=3) and the error bar shows a standard error thereof.

Fig. 5 shows result of Example 13. The longitudinal axis shows weight (mg/cm) per length of liquid that is pooled in a mouse enteric canal loop. The height of a column is a mean value of experiment values (n=3) and the error bar shows a standard error thereof.

Fig. 6 shows result of Example 14. The longitudinal axis shows radiation activity and the value is represented by 100% when vero toxin is not added. The height of a column is a mean value of experiment values (n=3) and the error bar shows a standard error thereof.

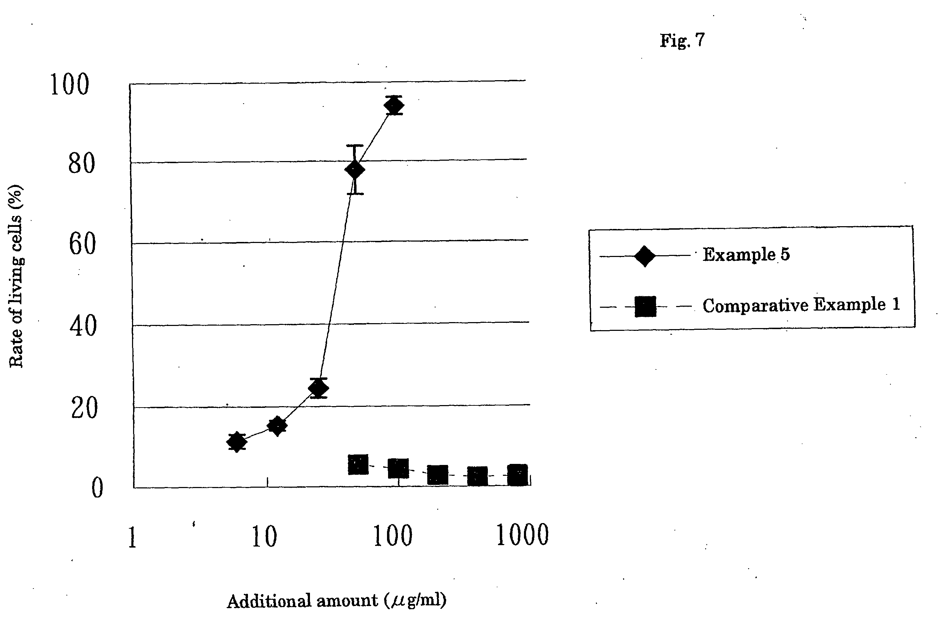

Fig. 7 shows result of Example 15. The longitudinal axis shows a rate of living cells and the horizontal axis shows the addition amounts (µg/ml) of materials obtained by the same method as in Example 5 or Comparative example 1. The addition amount of vero toxin is 62pg/ml at the final concentration. ◆ shows experimental result of the materials obtained by the same method as in Example 5, and ■ shows experimental result of the material obtained by the same method as in Comparative Example 1. The each value of the longitudinal axis shows a mean value of each experiment value (n=3) and the error bar shows a standard error thereof.

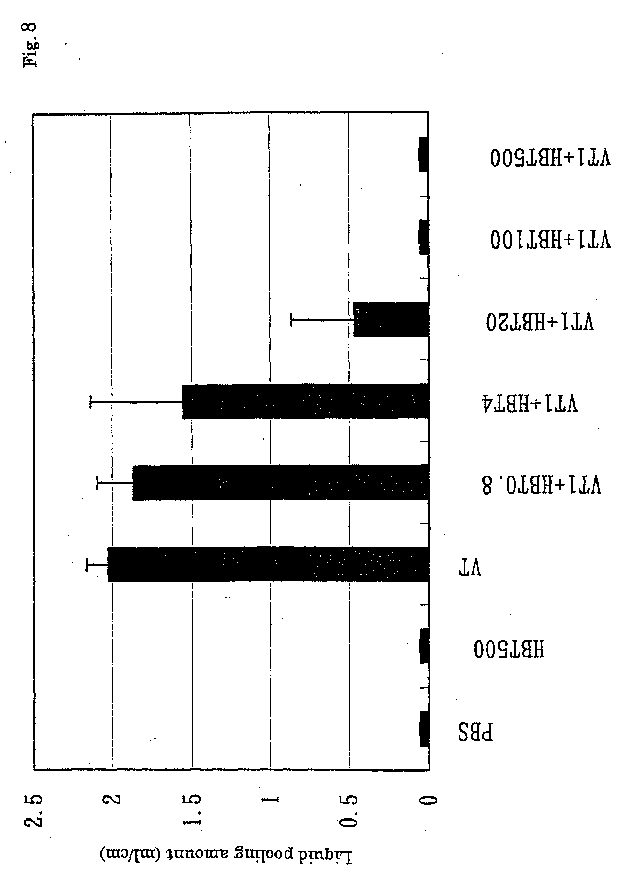

Fig. 8 shows result of Example 16. The longitudinal axis shows liquid weights per loop length (ml/cm). The horizontal axis shows experiment result. "PBS" shows the addition of PBS alone, "HBT 500" shows the addition of 500µg of materials obtained by the same method as in Example 5, "VT" shows the addition of 100ng of vero toxin alone, and "VT+HBT 0.8-500" shows the addition of 100 ng of vero toxin and 0.8-500µg of materials obtained by the same method as in Example 5. The values of the longitudinal axis shows mean values of each experiment value (n=3) and the error bar shows a standard error thereof.

### Best Mode for Carrying Out the Invention

As the raw materials of the proteotoxin neutralizer of the present invention, bine and bract parts of hop are preferably used. Particularly, whole parts can be used without separating each part of hop. The hop bracts mean that obtained by removing the lupulin part from hop cones. The hop bracts are obtained by removing the lupulin part after crushing and sieving the hop cones. However, in recent beer brewing, to omit the step of removing the hop bracts by sieving, without removing the hop bracts unnecessary in beer brewing, the hop bracts are formed into pellets. Such obtained hop pellets tend to use in beer brewing. Accordingly, as raw materials in the present invention, materials containing bine and bract parts of hop can be used without limit, and further hop cone and hop pellets containing hop bracts can be used as raw materials without problems.

As to a production method of the proteotoxin neutralizer, it comprises steps that raw materials containing hop bines, bracts, hop cones containing hop bracts, hop pellets, or these hop plant parts were extracted with water, organic solvent such as 80 v/v% or less alcohol, acetone, acetonitrile or the like which can be mixed with water. As a preferable example, a hydrous solution containing 50 v/v% or less ethanol may be used. The ratio of materials and extract solvent is preferably about 1:20-100 w/w, the extract temperature is 4-95°C. Preferably, the mixture is extracted under stirring for 20-60 minutes. The extract is obtained by filtration. If necessary, a filter aid such as pearlite may be used.

Solvent is removed from such obtained extract by using common methods such as concentration, freeze-drying or spray-drying, and powder of the proteotoxin neutralizer may be obtained. The resulting proteotoxin neutralizer is very useful and practical as it is, if necessary, it may be purified further by a method using absorbent resin. However, since this process aims to more purify, if unnecessary, it may be omitted.

The above extract is treated with granular synthetic resin having affinity for polyphenols to concentrate the proteotoxin neutralizer. This process may be conducted by passing the hop extract through a column packed with the granular synthetic resin, thoroughly washing the column, and eluting the neutralizer absorbed on the column. Otherwise, the granular resin may be immersed in the hop extract to conduct batch treatment.

When the proteotoxin neutralizer is absorbed on the synthetic resin, the hop extract is cooled to room temperature of 15-30°C, if necessary, for elevating the absorption efficiency, it is preferred that the concentration of organic solvent of the extract is previously lowered. As the synthetic resin, hydroxypropylated dextran, hydrophilicity vinyl polymer, styrene-divinyl benzene polymer and the like may be used.

The synthetic resin is then washed to further elevate the purity of the proteotoxin neutralizer. As the solvent for washing, preferably water or a 1-10 w/w% aqueous ethanol solution may be used. The solvent amount is preferably about 1-10 times of the resin amount to wash the neutralizer.

The proteotoxin neutralizer is then eluted to remove from the synthetic resin that the polyphenols are absorbed. As the solvent used for the elution, hydrous alcohol, hydrous acetone, hydrous acetonitrile and the like may be used. Particularly preferable solvent is 30 w/w% or more aqueous ethanol solution or ethanol. The amount of the eluate passing through the resin is preferably about 2-6 times of the amount of the resin.

The solvent is removed from the resulting elute by using common methods such as concentration, freeze-drying or spray drying, and powder of the proteotoxin neutralizer may be obtained. At the vacuum concentration, alcohol; acetone, acetonitrile and the like may be recovered to recycle. Used synthetic resin is washed with an aqueous solution of 80 v/v% or more alcohol or about 0.05 N sodium hydroxide and the like, and the resin may be repeatedly recycled.

Such obtained proteotoxin neutralizer may be used as it is, further the purity may be raised by a method using a ultrafilter film as described in the following. This process is done for raising the purity of the neutralizer, if necessary, it can be omitted.

The proteotoxin neutralizer prepared by the above method is dissolved in water or organic solvent mixable with water, and treated with a ultrafilter film having the fractional molecule of 1,000 or more. The raw materials of the ultrafilter film such as cellulose, cellulose acetate, polysulfone, polypropylene, polyester, polyethersulfone and PVDF and the like may be used without any restriction. The ultrafilter film having the fractional molecule of 1,000 or more can be used without any problem. However, when the ultrafilter film having too high fractional molecule weight is used, the yield is lowered extremely. When the molecule weight is low, the treatment time becomes long. Preferable fractional molecule weights of the ultrafilter film are about 5,000-50,000. Although the treatment depends on the kind of solvent, or the ratio of extract solvent to hop or hop bracts, it is preferably done until the amount of the upper rest liquid is lowered to about 1/10-1/100 as compared with the beginning of the treatment. Although the pressure depends on the ultrafilter film or filter device, it is preferably 0.1-10.0 kg/cm². If necessary, the upper rest liquid treated may be diluted with a suitable solvent again, and recycled to raise the purity.

The solvent or the resulting upper rest liquid is removed by using common methods such as concentration, freeze-drying or spray-drying, and powder of the proteotoxin neutralizer may be obtained. At the vacuum concentration, alcohol, acetone, acetonitrile and the like may be recovered and reused.

Thus obtained proteotoxin neutralizer is odorless powder having slight bitter taste and skin color, brown color or pale yellow color. The neutralizer adsorbs to synthetic resin having the affinity for polyphenol, and it is proanthocyanidins that does not penetrate the ultrafilter film having a fractional molecule weight of 1,000 or more.

The yield is 0.5-20.0w/w% converted to a weight of hop bracts, and 0.5-15.0w/w% converted to a weight of hop cone.

The resulting proteotoxin neutralizer can be formulated with usually used carriers, aids, additives and the like. The neutralizer can be used as medical supplies orally or parenterally or as food and drink by mixing with food materials.

Oral medical supplies are tablets, capsules, granule, syrup and the like, and parenteral medical supplies are external medicines such as ointment, cream and solution, and injectables such as sterility solutions and suspension. When these supplies are administered to human bodies, the administration amounts are 2-1,000mg per day and 2-500mg per one or few times per day to give sufficient effects.

The medical supplies containing the proteotoxin neutralizer of the present invention can be formulated by a desired unit volume form along with pharmaceutically approved vehicles, carriers, fillers, binders, stabilizers, flavors and the like. Adjuvants mixable with tablets or capsules are as follows: binders such as tragacanth, arabic gam, corn starch and gelatin, fillers such as microcellulose, swelling agents such as corn starch, all geratinization starch, alginic acid, lubricants such as magnesium stearate, sweetening agents such as sucrose, nipple and saccharin, and flavors such as peppermint, akamono oil and cherries. The capsules can contain liquid carriers such as fats and fatty oils in addition to the above materials. The other materials are coating agents. Further, the physical forms of formulations can be changed by the other methods. As examples, tablets can be coated with schellac or sucrose. Syrup or elixir can contain sucrose as a sweetening agent, methyl or propylparaben as an antiseptic, a pigment and a flavor such as a cherry or orange flavor.

Sterilized constitution for injection can be formulated by common methods that active materials in vehicles such as water for injection, natural plant oils such as sesame oil, coconut oil, peanut oil and cotton seed oil, or synthetic fatty vehicles such as ethyl oleate are dissolved or suspended. Moreover, if necessary, buffer, antiseptic, antioxidant and the like can be formulated. External medicines such as ointment or cream can be obtained by common methods using Vaseline, paraffin, fats and fatty oils, lanolin, macrogol and the like.

Food and drink containing the proteotoxin neutralizer of the present invention may be formed by the above formulation. Otherwise, it may be processed by general methods by adding requirement into food materials of wheat gluten, Japanese cracker, cookies or drinks. The neutralizer is processed and orally taken as health foods or functionality foods of 5-500mg per day by dividing into a few times for ill prevention and health maintenance.

When the proteotoxin neutralizer of the present invention is added to these food and drink, it is desired that the neutralizer may be added as powder, in the final concentration of 1-500ppm, and preferably, 10-300ppm in a 1-2% aqueous solution or aqueous alcohol solution or alcoholic solution.

### Example

Although the following examples describe in detail the embodiment of the present invention, the present invention is not limited into these examples.

### Example 1 (Preparation of the proteotoxin neutralizer from hop cone by using a synthetic adsorbent of a gel type)

Hop cone 20g were crushed in a mortar, and extracted with water 2L by stirring at a temperature of 95°C for 40 minutes. After filtration, extracted water was cooled, passed through a column filled with hydrophilic vinyl polymer resin 80ml, and washed with a 5% ethanol aqueous solution 400ml. 80% ethanol aqueous solution was passed through the column, the eluted solution was recovered and freeze-dried, and the proteotoxin neutralizer 800mg was obtained as pale yellow odorless powder having faint bitter taste. The yield from the hop was 4%.

### Example 2 (Preparation of the proteotoxin neutralizer from hop bracts by using a synthetic adsorbent of a gel type)

Hop bracts 20g were extracted with 50% ethanol aqueous solution 600ml by stirring at a temperature of 30°C for 20 minutes. After filtration, extracted liquid was vacuum concentrated, and the concentrated liquid was passed through a column filled with styrene-divinylbenzene resin 80ml and washed with water 400ml. 80% ethanol aqueous solution 400ml was passed through the column, the eluted solution was recovered and freeze-dried, and the proteotoxin neutralizer 1.6g was obtained as pale yellow odorless powder having faint bitter taste. The yield from the hop bracts was 8%.

### Example 3 (Preparation of the proteotoxin neutralizer from hop cone by using a ultrafilter film)

Hop cone 20g were crushed in a mortar, and extracted with water 2L by stirring at a temperature of 95°C for 40 minutes. After filtration, extracted liquid was cooled, passed through a ultrafilter film having fraction molecular weight 50,000 at 1.0kg/cm² at room temperature to obtain the liquid 20ml. The resulting upper rest liquid was vacuum-dried, and the proteotoxin neutralizer 200mg was obtained as pale yellow odorless powder having faint bitter taste. The yield from the hop was 1%.

### Example 4 (Preparation of the proteotoxin neutralizer from hop bracts by using a ultrafilter film)

Hop bracts 20g were extracted with a 50% ethanol aqueous solution 600ml by stirring at a temperature of 80°C for 40 minutes. After filtration, extracted liquid was passed through a ultrafilter film having fraction molecular weight 10,000 at 3.0kg/cm² at room temperature to obtain the liquid 60ml. The resulting upper rest liquid was freeze-dried, and the proteotoxin neutralizer 0.8g was obtained as pale yellow odorless powder having faint bitter taste. The yield from the hop bacts was 4%.

### Example 5 (Further purification and qualitative analysis of the proteotoxin neutralizer)

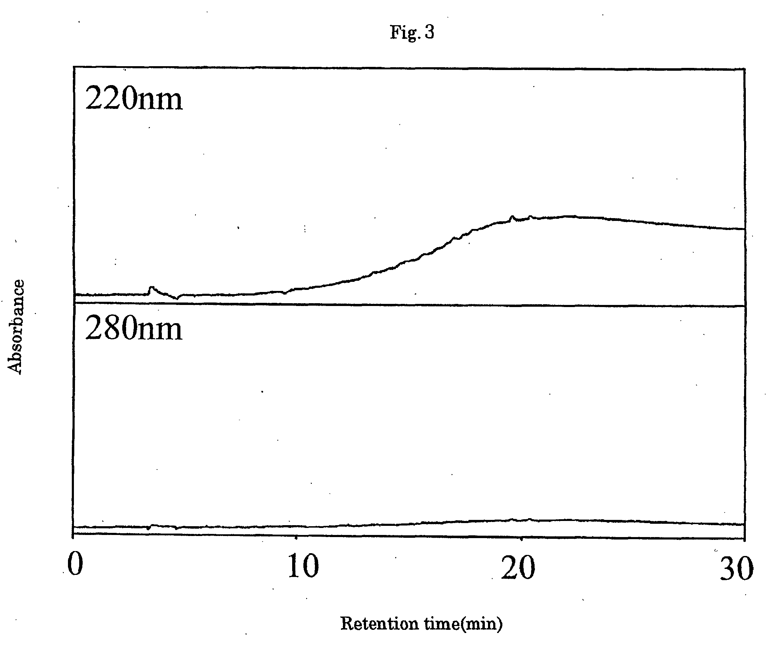

The proteotoxin neutralizer 0.8g obtained in Example 2 was dissolved in 10% ethanol aqueous solution 500ml, and treated with a ultrafilter film having fraction molecular weight 5,000 at 1.0kg/cm² at room temperature to obtain the liquid 20ml. The resulting upper rest liquid was freeze-dried, and the proteotoxin neutralizer 0.4g was obtained as odorless powder having faint bitter taste and a skin color. The powder was analyzed by HPLC by using the following conditions, and it had a chromatogram as shown in Fig. 3. The powder was analyzed by catechin determination (a food official analysis) which is one of general quantitative analysis of polyphenols, and the neutralizer was 40.6% calculated in terms of catechin content.

(HPLC conditions) device: Shimazu LC-10A system, column:ODS-80TM (Toso, 4.6mm I.D.×25cm), mobile phase: linear gradient (A liquid:B liquid)=from (100:0) to (50:50) for 30 minutes, A liquid:5% acetonitrile (containing 0.1°/ HCl), B liquid: acetonitrile, sample injection amount: 20µg, detection: multiwavelength was detected at 200-300nm.

### Example 6 (Tablets and capsules)

| | |
|---|---|
| Material obtained as in Example 5 | 10.0g |
| Lactose | 75.0g |
| Magnesium stearate | 15.0g |
| Total | 100.0g |

The above components were uniformly mixed, and tablets and capsules were obtained by a general method. Instead of the material obtained by the method described in Example 5, by the same method, tablets and capsules were formed from materials obtained by the methods described in Examples 1,2,3 and 4, respectively.

### Example 7 (powder and granule)

| | |
|---|---|
| Material obtained as in Example 5 | 20.0g |
| Starch | 30.0g |
| Lactose | 50.0g |
| Total | 100.0g |

The above components were uniformly mixed, and powder and granule were obtained by a general method. Instead of the material obtained by the method described in Example 5, by the same method, powder and granules were formed from materials obtained by the methods described in Examples 1,2,3 and 4, respectively.

### Example 8 (injection)

| | |
|---|---|
| Material obtained as in Example 5 | 1.0g |
| Detergent | 9.0g |
| Physiological saline | 90.0g |
| Total | 100.0g |

The above components were heated, mixed and sterilized, and injection was obtained. Instead of the material obtained by the method described in Example 5, by the same method, injection was produced from materials obtained by the methods described in Examples 1,2,3 and 4, respectively.

### Example 9 (wheat gluten)

| | |
|---|---|
| Sucrose | 20.0g |
| Thick malt syrup (solid content 75%) | 70.0g |
| Water | 9.5g |
| Coloring agent | 0.45g |
| Flavor | 0.045g |
| Material obtained as in Example 5 | 0.005g |
| Total | 100.0g |

Using each component having the above amount, wheat gluten was made by a general method. Instead of the material obtained by the method described in Example 5, by the same method, wheat gluten was produced from materials obtained by the methods described in Examples 1,2,3 and 4, respectively.

### Example 10 (juice)

| | |
|---|---|
| Concentrated orange fruit juice | 15.0g |
| Fructose | 5.0g |
| Citric acid | 0.2g |
| Flavor | 0.1g |
| Coloring agent | 0.15g |
| Sodium ascorbic acid | 0.048g |
| Material obtained as in Example 5 | 0.002g |
| Water | 79.5g |
| Total | 100.0g |

Using each component having the above amount, juice was made by a general method. Instead of the material obtained by the method described in Example 5, by the same method, juice was produced from materials obtained by the methods described in Examples 1,2,3 and 4, respectively.

### Example 11 (cookies)

| | |
|---|---|
| Soft flour | 32.0g |
| Whole egg | 16.0g |
| Butter | 16.0g |
| Sucrose | 25.0g |
| Water | 10.8g |
| Baking powder | 0.198g |
| Material obtained as in Example 5 | 0.002g |
| Total | 100g |

Using each component having the above amount, cookies were made by a general method. Instead of the material obtained by the method described in Example 5, by the same method, cookies were produced from materials obtained by the methods described in Examples 1,2,3 and 4, respectively.

### Comparative example 1

With reference to a method of Hattori et al (M. Hattori et al., Chem. Pharm. Bull., 38, 717-720 (1990)), a polyphenol fraction was prepared from green tea. Green tea 10g from Shizuoka prefecture in Japan was extracted with boiled water 200ml. The extracted liquid was freeze-dried (2.7g), dissolved in 30% methanol, and passed through ODS column (15mm I.D.×10cm). The eluate was freeze-dried to obtain a polyphenol fraction 2.4g as yellow powder.

### Example 12 Inhibiting effect of ADP ribosyltransferase action of cholera toxin

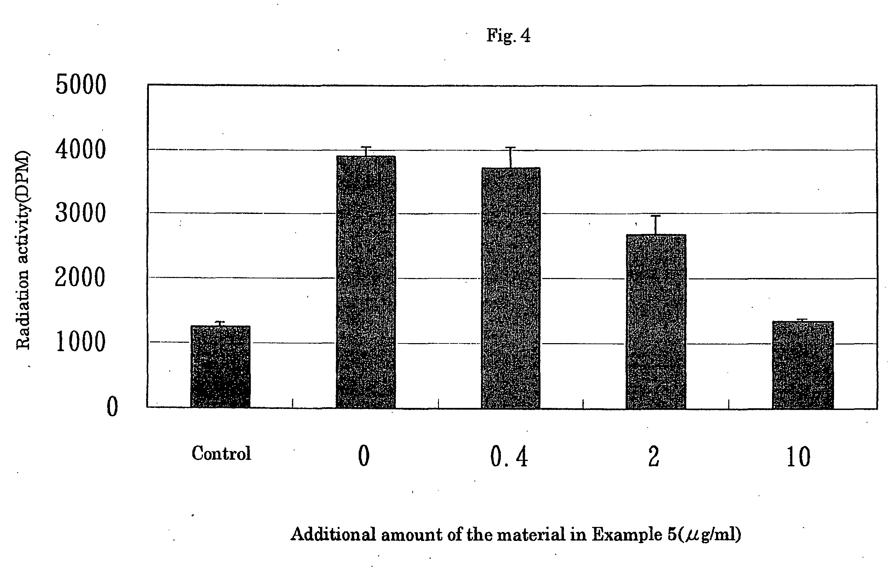

The assay was conducted by a Noda et al's method (Noda M. et al., Biochemistry, 28, 7936 (1989)). Firstly, a cholera toxin solution having the following composition and an NAD reaction solution were prepared. Mixture of this cholera toxin solution 20µl, PBS solution 20µl having several concentrations of the material obtained as shown in Example 5, and distilled water 60µl were prepared. To this mixture, the NAD reaction solution 100µl was added. The mixture was then incubated at a temperature of 30°C for 90 minutes. After the incubation, the reaction solution 150µl was collected and passed through Dowex AG1-X2 (Bio-Rad Inc.) of a 0.5cm × 4 cm column to remove unreacted adenine ¹⁴C NAD. The amount of the resulting adenine ¹⁴C NAD-ribosylation agmatine was determined by a liquid scintillation counter (Beckman LS6500). The amount of adenine ¹⁴C NAD-ribosylation agmatine was used as an indicator to determine ADP-ribosyltransferase activity. The result is shown in Fig. 4. The material obtained as shown in Example 5 neutralized the ADP-ribosyltransferase action of the cholera toxin in concentration dependence.

The composition of the cholera toxin solution: 1mg/ml cholera toxin 180µ 1, 1M potassium phosphate buffer (pH 7.5) 60µl, 1M dithiothreitol aqueous solution 120µl and distilled water 840µl were mixed and incubated at a temperature of 37°C for 20 minutes to obtain the cholera toxin solution.

The composition of the NAD reaction solution: 1M potassium phosphate buffer (pH 7.5) 190µl, 0.1M MgCl₂ aqueous solution 190µl, 1M dithiothreitol aqueous solution 76µl, 10mg/ml ovalbumin 38µl, 1M agmatine 76µl, ¹⁴C NAD (25uCi/ml) 19µl and distilled water 1311µl were mixed to obtain the ¹⁴C NAD reaction solution.

### Example 13 Neutralizing effect of liquid accumulation toxin of cholera toxin in mouse enteric canals

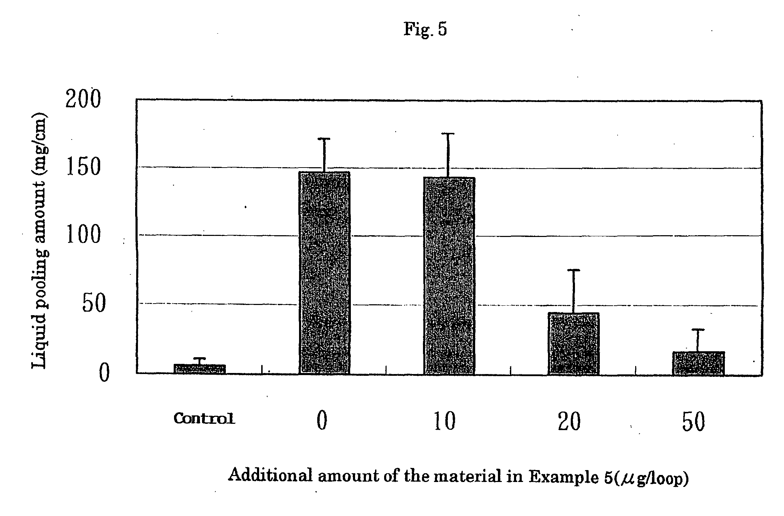

Female ddY mice of 5-6 weeks having weight 19-21g were treated by celiotomy under anesthesia. The ileum parts of about 5cm were linked to make loops of a bag type. A certain amount (2µg) of cholera toxin and a solution 200µ 1 containing each concentration of the material obtained as shown in Example 5 were injected in the loops. The abdomen of the mice were closed and kept quiet. Then, the mice were euthanized and the weight of the liquid accumulated in the loops and the loop length were determined. Liquid weight per loop length (mg/cm) was calculated to index the toxicity of cholera toxin. The result was shown in Fig. 5. The material obtained as shown in Example 5 neutralized the liquid accumulation of the cholera toxin in concentration dependence.

### Example 14 Inhibiting effect of RNA N-glycosidase action of vero toxin

A certain concentration (240nM) of a vero toxin aqueous solution 5µl and 240-2400µg/ml of an aqueous solution of the material obtained as shown in Example 5 were mixed and permitted to stand for one hour at room temperature. Then, 50µl of liquid dissolving rabbit reticulocytes was prepared as shown in the following constitution, this liquid was added to the mixture, and protein synthesis reaction was conducted on a water bath at a temperature of 30°C for 20 minutes. After the reaction, the sample was moved on ice, an aqueous solution 1ml of 10% trichloroacetic acid was added, and the mixture was boiled on boiled water for 10 minutes, and the reaction was stopped. After the sample was cooled with ice, the synthesized protein was adsorbed on a nitrocellulose filter and washed with a 10% trichloroacetic acid aqueous solution 1ml two times. After drying the protein, the radiation activity was determined with a scintillation counter.

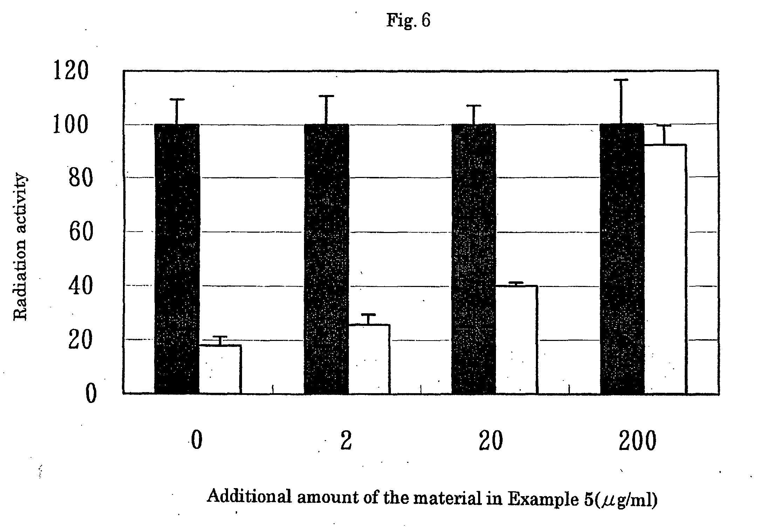

The result was shown in Fig. 6. The materials obtained as shown in Example 5 suppressed dependently in the concentration the protein synthesis with the vero toxin. It shows that the materials inhibited the RNA N-glycosidase action based on the vero toxin.

Composition of the liquid dissolving rabbit reticulocytes: 50mM adenosin-3 phosphate aqueous solution 15 µl, 10mM guanosine-3 phosphate aqueous solution 15µl, 300mM creatine phosphate aqueous solution 30µl, 1 mg/ml creatine kinase aqueous solution 10µl, 1M dithiothreitol aqueous solution 4µl, 1M HEPES buffer (pH7.5) 10µl, 3M potassium chloride aqueous solution 15 µl, 100mM magnesium acetate aqueous solution 15µl, an aqueous solution 15µl containing each 5mM amino acid except Leu, 100uCi/ml ¹⁴C-Leu aqueous solution 50µl, 4mg/ml hemin aqueous solution 4µl and rabbit reticulocytes hemolytic liquid 500µl.

### Example 15 Effect of neutralization of cell toxin of vero toxin to vero cells

Previously, vero cell suspension 100µl adjusted to 2.0 × 10⁵ cells/ml was pipetted into a 96 hole plate, and permit to stand one night to prepare unit layer membrane of vero cells. The vero toxin of a certain concentration and 10µl of PBS solution total 80µl containing the materials of every kind which was obtained as shown in Example 5 and Comparative example 1 were added to the holes, and the mixture was cultured in a 5% CO₂ incubator at a temperature of 37°C for 48 hours. After the culture, the survival rate of vero cells was determined with Cell Counting Kit (Dojin Kagaku Co.). The results are shown in Fig. 7. The materials obtained as shown in Comparative example did not show any effect, but the materials obtained as shown in Example 5 neutralized concentration-dependently the cell toxicity to vero cells of vero toxin.

### Example 16 Effect of neutralization of liquid accumulating toxin of vero toxin in the rabbit enteric canal

Male Nippon white rabbits of about 2 kg weight of 11-13 weeks were treated by celiotomy under anesthesia. The ileum parts of about 10cm were linked to make loops of a bag type. A certain amount of vero toxin and a PBS solution 1ml containing each concentration of the material obtained as shown in Example 5 were injected in the loops. The abdomen of the rabbits was closed and kept quiet a whole day and night. Then, the rabbits were euthanized and the weight of the liquid accumulated in the loops and the loop length were determined. Liquid weight per loop length (mg/cm) was calculated to index the toxicity of vero toxin. The result was shown in Fig. 8. The material obtained as shown in Example 5 neutralized the cell toxicity of vero toxin in concentration dependence.

### Industrial Applicability

The proteotoxin neutralizer of the present invention contains proanthocyanidins obtained from hop as effective components. The agent neutralizes the cell toxin having ADP-ribosyltransferase action and RNA N-glycosidase action, thereby it shows excellent effect for prevention and treatment of the infectious diseases based on the bacilli producing the ADP-ribosyltransferase or the bacilli producing the RNA N-glycosidase.

## Claims

1. A proteotoxin neutralizer which contains proanthocyanidins obtained from hop as effective components.

2. A proteotoxin neutralizer which contains proanthocyanidins obtained from hop bracts as effective components.

3. The neutralizer according to claim 1 or 2 wherein the proanthocyanidins do not pass through an ultrafilter having fraction molecules of 1000 or more.

4. The neutralizer according to claim 3 wherein the proteotoxin has RNA N-glycosidase activity or ADP-ribosyltransferase activity.

5. The neutralizer according to claim 4 wherein the proteotoxin is vero toxin or cholera toxin.
